# EUROPEAN PATENT APPLICATION

(11) **EP 1 593 396 A1**
(43) Date of publication of application: **09.11.2005**
(21) Application number: 05076692.2
(22) Date of filing: 26.01.2001
(51) Int. Cl.: A61L 2/22, A01N 59/00, A01N 25/06

(54) **Antimicrobial flash-dry disinfectant aerosol**

(30) Priority: 26.01.2000 CA 2297002
(62) Divisional of application: 01902209.4
(71) Applicant: Triosyn Holding Inc., Mirabel, Québec J7J 1M3 (CA)
(72) Inventor: Messier, Pierre Jean, St-Sauveur Quebec J0R 1R7 (CA); Tanelli, Joe, St-Leonard Quebec H1R 1S6 (CA); St.-Louis, Jean-Pierre, Prevost Quebec J0R 1T0 (CA); Bourget, Stephane, Montreal Quebec H2P 2K3 (CA)
(74) Representative: Beresford, Keith Denis Lewis

(57) **Abstract**

A liquid flash-dry aerosol disinfectant composition comprising a flash vaporisation component and an effective amount of an antimicrobial agent, said flash vaporisation component being able, once the flash-dry disinfectant composition is sprayed in aerosol form onto a surface, to flash vaporise so as to leave an essentially dry surface having antimicrobial agent deposited thereon.

## Description

The present invention relates to an aerosol apparatus (e.g. pressurised) for dispensing an antimicrobial agent in an aerosol spray onto a surface to be disinfected. The aerosol apparatus may contain a flash-dry disinfectant composition comprising said antimicrobial agent (e.g. the antimicrobial agent may be hydrogen peroxide).

Disinfectant compositions are known which comprise an alcohol(s) and hydrogen peroxide, compositions which have a bactericidal effect when applied to a surface (please see U.S. patent nos 5,916,568 and 5,868,998 which describe the use of hydrogen peroxide as a bactericide, the entire contents of these patents are incorporated herein by reference).

It would be advantageous to have a means for facilitating the application of or dispensing of a disinfectant compositions onto a surface so as to facilitate the antimicrobial effect of such disinfectant composition while at the same time being able to facilitate or accelerate the drying of the surface to be disinfected.

The present invention provides in one aspect thereof in an aerosol spray apparatus, for applying an antimicrobial agent to a surface to be disinfected, comprising an aerosol spray nozzle coupled to a container means comprising gas propellant means (e.g. a freon, nitrogen, air, carbon.dioxide, etc.) and means for containing a liquid disinfectant composition able to be sprayed from the apparatus, said aerosol apparatus being configured for dispensing said disinfectant composition through said spray nozzle, under pressure due to the presence of the gas propellant means, as an aerosol spray onto said surface to be disinfected, the improvement wherein the liquid disinfectant composition is a liquid flash-dry disinfectant composition comprising (e.g. consisting of) a flash vaporisation component and an effective amount of an antimicrobial agent, said flash vaporisation component being able, once the flash-dry disinfectant composition is sprayed from the apparatus, to flash vaporise (e.g. at ambient temperature and pressure) so as to leave an essentially dry surface having antimicrobial agent (e.g. hydrogen peroxide) deposited thereon.

In accordance with a further aspect the present invention provides a liquid flash-dry aerosol disinfectant composition comprising (e.g. consisting of) a flash vaporisation component and an effective amount of an antimicrobial agent, said flash vaporisation component being able, once the flash-dry disinfectant composition is sprayed in aerosol form onto a surface, to flash vaporise (e.g. at ambient temperature and pressure) so as to leave an essentially dry surface having antimicrobial agent (e.g. hydrogen peroxide) deposited thereon.

In accordance with another aspect the present invention provides a method for disinfecting a surface comprising applying a liquid flash-dry disinfectant composition as an aerosol spray onto such surface, said liquid flash-dry aerosol disinfectant composition comprising (e.g. consisting of) a flash vaporisation component and an effective amount of an antimicrobial agent, said flash vaporisation component being able, once the flash-dry disinfectant composition is sprayed in aerosol form onto a surface, to flash vaporise (e.g. at ambient temperature and pressure) so as to leave an essentially dry surface having antimicrobial agent (e.g. hydrogen peroxide) deposited thereon.

In accordance with the present invention the components of the flash-dry disinfectant composition (i.e. the antimicrobial agent and the flash vaporisation component) may be mixed together just prior to the flash-dry disinfectant composition being applied to a surface.

Accordingly the present invention also provides in an aerosol spray apparatus, for applying an antimicrobial agent to a surface to be disinfected, comprising an aerosol spray nozzle coupled to a container means comprising gas propellant means, said aerosol apparatus being configured for dispensing a liquid disinfectant composition through said spray nozzle, under pressure due to the presence of the gas propellant means, as an aerosol spray onto said surface to be disinfected, the improvement wherein said liquid disinfectant composition is a liquid flash-dry disinfectant composition comprising (e.g. consisting of) a flash vaporisation component and an effective amount of an antimicrobial agent element, said flash vaporisation component being able, once the flash-dry disinfectant composition is sprayed from the apparatus, to flash vaporise so as to leave an essentially dry surface having antimicrobial agent deposited thereon, wherein said container means comprises a first container means containing said antimicrobial agent and a second container means containing said flash vaporisation component and wherein said aerosol spray apparatus comprises mixing means for mixing said antimicrobial agent and said flash vaporisation component together prior to dispensing said liquid flash-dry disinfectant composition through said spray nozzle.

In accordance with the present invention the antimicrobial agent may, for example, be hydrogen peroxide; however any other suitable (known) antimicrobial agent able to be incorporated into a formulation able to be disposed on a surface by means of an aerosol spray may be used.

The flash vaporisation component may, for example, comprise an alkanol of formula ROH wherein R is a group containing from 1 to 6 carbon atoms such as for example ethanol, isopropanol, butanol and the like). Additionally the flash vaporisation component may, for example, comprise a ketone of up to six carbon atoms (e,g, acetone); an ether of up to six carbon atoms; a halogenated organic compound such as, for example, freon, chloroform; etc..

In accordance with the present invention a flash-dry disinfectant composition (in a container means or as being sprayed as an aerosol onto the surface to be disinfected) may for example comprise (e.g. consists of) 1 to 50 % (e.g. 3 to 30%) or more (volume/volume) antimicrobial agent (for example, 3 to 30% or more (volume/volume) hydrogen peroxide), 10 to 90% (e.g. 10 to 85 %) or more (volume/volume) flash vaporisation component (for example 10 to 85 % or more (volume/volume) of a flash vaporisation component comprising an alcohol such as for example a flash vaporisation component which is ethanol). The flash-dry disinfectant composition may, for example, comprise, a remainder component or element which may, for example, comprise or be water.

In accordance with the present invention a flash-dry disinfectant composition (in a container means or as being sprayed as an aerosol onto the surface to be disinfected) may for example in particular comprise (e.g. consists of) 3 to 30% by volume hydrogen peroxide, 10 to 85 % by volume of an alcohol such as for example ethanol and 10 to 65 % by volume of water.

More particularly, the flash-dry disinfectant composition of the present invention may comprise any (liquid) flash vaporisation component which is able to impart to the composition a flash vaporisation characteristic i.e. to achieve in a relatively, (desired or necessary) short period of time after applying the flash dry aerosol to a surface, a state wherein the surface is essentially dry leaving behind the antimicrobial agent (i.e. a surface has achieved a desired or necessary state of dryness). The flash dry liquid may for example be one which is able to evaporate at a relatively rapid rate at ambient room temperature and pressure, i.e. having a high volatility (e.g at 15 degrees centigrade or higher such as at 20 to 25 degrees centigrade). The flash-dry disinfectant composition may for example, comprise a flash vaporisation component comprising one or more of the above mentioned materials; it may for example comprise an alkanol comprising from 2 to 5 carbon atoms (e.g ethanol, isopropanol, butanol etc...), an alkoxy substituted alkane (i.e. an ether) comprising for 2 to 4 carbon atoms (e.g. diethyl ether - ethoxy ethane, ....); etc... The flash dry materials may be obtained form any known suppliers. An alcohol for example may be obtained from any known supplier such as Fisher Scientific, Signa-Aldrich (e.g. as a composition comprising 82-95% by volume ethanol, 2 to 10% by volume water and 3 to 10% by volume methanol).

The antimicrobial agent may as mentioned above be hydrogen peroxide; it may also be a compound that forms peroxide in an aqueous medium (i.e. in situ). The hydrogen peroxide may be obtained from any known supplier such as Fisher Scientific, Signa-Aldrich (e.g. as a composition comprising 30% by volume hydrogen peroxide and 70% by volume water).

The aerosol apparatus may take any desired (known) form provided that it may deliver the flash-dry disinfectant composition to a surface in aerosol (e.g. in micro aerosol).

The aerosol apparatus may for example comprise a pressurised container containing a pre-dosed amount of inert propellant gas component in addition to the flash-dry disinfectant composition. In this case, the basic aerosol container is configured to be able to contain or store the flash-dry disinfectant composition under pressure which is due to the additional presence in the container of an inert pressurising gas component such as air, nitrogen gas, etc.. Alternatively the basic aerosol container may comprise there within or otherwise be associated with a secondary pressurized gas container which is appropriately coupled in any suitable (known) manner to the spray nozzle so as to be able to induce the disinfectant composition through the nozzle as an aerosol spray. The propellant gas may be any gas which is at least essentially inert with respect to the flash-dry disinfectant composition components (e.g. nitrogen gas, air, and the like); it may also be a freon gas.

The aerosol apparatus may alternatively, may comprise a mechanism for manually introducing atmospheric air into the container so as to generate a positive pressure therein as mentioned above; an example of this latter type of aerosol apparatus is described in U.S. patent no. 5,265,775, (the entire contents of this patent is incorporated herein by reference.

The aerosol apparatus may for example be configured in any known manner so as to be able to generate or provide a spray having droplet sizes of for example from 1 to 100 microns; larger sized droplets may of course be used provided that the desired flash dry effect is achieved when the composition is applied to a surface to be disinfected.

The aerosol container may have any type of known spray nozzle system coupled to the pressurised container for dispensing an aerosol spray or jet onto a surface. The nozzle is of course configured so as to be able to pass from a normal closed configuration (no spray being developed) to an active open configuration, the opened configuration being such so as to allow the internal positive pressure persisting in the aerosol container to urge the flash-dry disinfectant composition out of the container as an aerosol spray. Suitable (aerosol) type may be obtained from Electron Microscopy Sciences, Pennsylvania USA (i.e. Spray Safe Atomizer (64186); from Four Star Chemicals, ,California USA (i.e Aluminum and epoxy coated cans); and from CEODEUX Firetec, out of Luxembourg (i.e. 3-Six and nine liter extinguisher type sprayer).

In the drawings which illustrate example embodiments of the present invention :
- Figure 1: is a schematic illustration of a known type of aerosol can containing a flash dry disinfection composition of the present invention;
- Figure 2: is a schematic illustration of an alternative type of aerosol can containing separated liquid components of a flash dry disinfection composition of the present invention; and
- Figure 3: is a is a schematic illustration of an additional type of (known) aerosol can containing separated liquid components of a flash dry disinfection composition of the present invention.

With respect to the drawings the same reference numerals will be used to designate the same element(s).

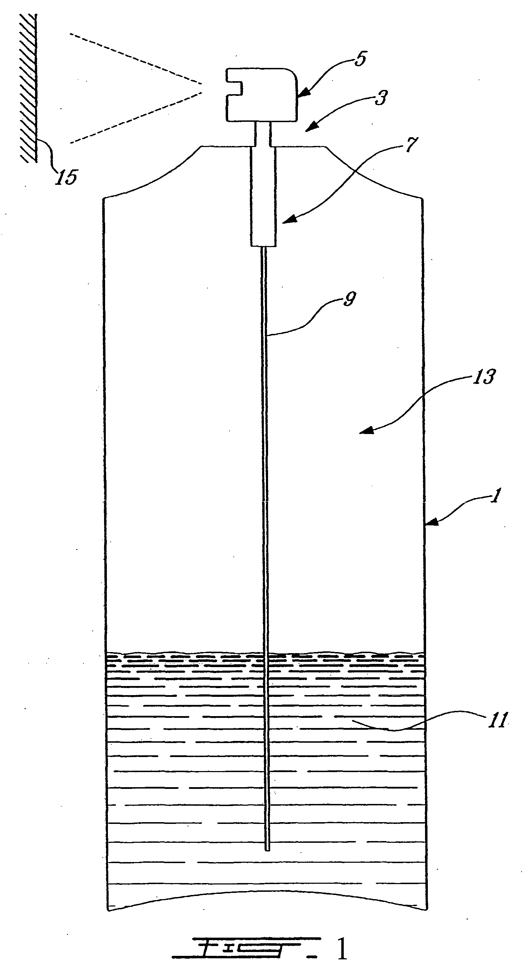

Referring to figure 1 the aerosol spray can comprises an outer cylindrical container shell 1, a spray valve nozzle assembly 3 (comprising a spray nozzle 5) and a siphoning assembly 7 (including a siphoning tube 9). The spray can also contains therein a liquid flash-dry disinfection composition 11 in accordance with the present invention. A pressure space 13 is located above the disinfection composition 11 and may contain a pressurised gas for inducing the disinfection composition to pass up through the siphoning assembly tube 9 to the spray nozzle 5 for ultimately being passed therethrough as a spray aerosol indicated in dotted outline; the spray of course being direct to the surface 15 to be sterilized.

In accordance with the embodiment illustrated in figure 1 the various liquid component forming the disinfectant composition may be poured into the container shell 1; the valve nozzle assembly 3 is then used to cap or seal (in a permanent or removable manner) the container shell 11 in gas or air tight fashion; and then the container is pressurized (through the nozzle assembly 3 - nozzle 5 removed) using an inert gas to a pressure ranging for example from 60 psig to 200 psig. Alternatively, instead of an inert gas the container once the nozzle assembly is in place may be pressurized by being filled with a suitable (known) low boiling point refrigerant gas such as a freon.

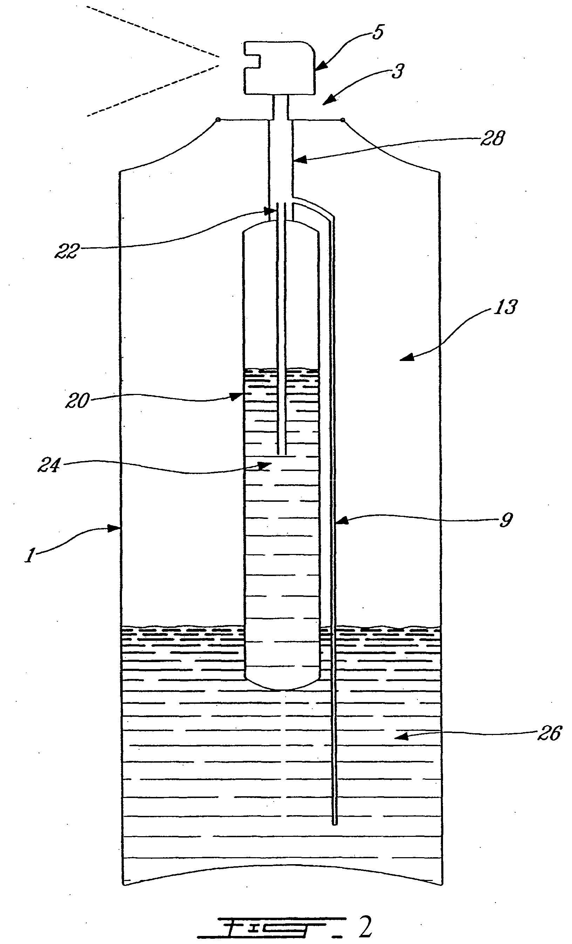

Turning to figure 2, this figure illustrates an aerosol spray can assembly wherein components of the flash-dry disinfectant composition are kept apart in separate container means until the time at which they are to be sprayed. This can assembly also comprises an outer cylindrical container shell 1, a spray valve nozzle assembly 3 (comprising a spray nozzle 5) and a siphoning tube 9. The spray can however, also has an inner container 20 which is also provided with a siphon tube assembly indicated generally by the reference numeral 22. The inner container 20 contains a first liquid component 24 of the flash-dry disinfection composition whereas a second liquid component (indicated by the reference numeral 26) is located outside the inner container 20 but inside the container shell 1. The can assembly is also provided with a siphoning and mixing assembly 28. A pressure space 13 is located above the second disinfection component 26 and may contain a pressurised gas for inducing the first and second liquid components 24 and 26 to pass up through respective siphoning assembly tubes 9 and 22 to the mixing assembly 28 and then on to the spray nozzle 5 for ultimately be passed therethrough as a spray aerosol indicated in dotted outline. The system shown may be pressurised through the nozzle assembly 3 (first removing the nozzle 5) In this case when nozzle 5 is depressed both liquid components 24 and 26 will be forced into the mixing assembly, mixed and then sprayed out the nozzle 5.

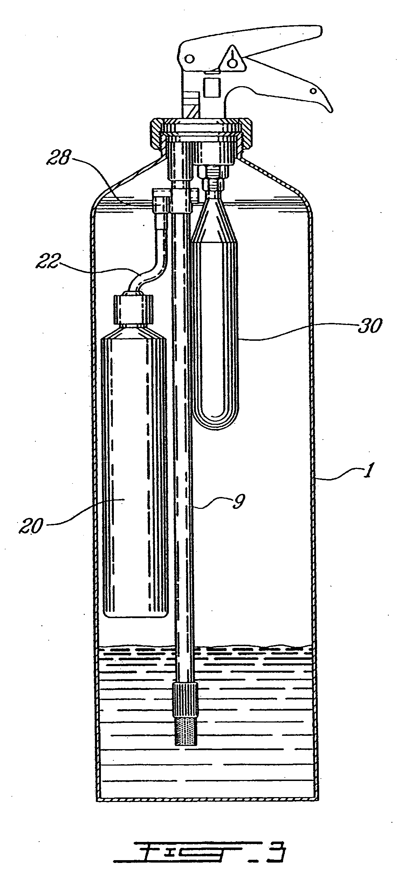

Referring to figure 3, this figure illustrates an alternate aerosol spray can assembly wherein components of the flash-dry disinfectant composition are kept apart in separate container means until the time at which they are to be sprayed; the spray container may be obtained from CEODEUX Firetec, out of Luxembourg. This assembly differs from that shown in figure 2 in that the pressurization is accomplished by use of a sealed pressurized CO₂ canister 30. In this case when the respective nozzle is initially depressed the carbon dioxide in the canister 30 is allowed to escape (in known fashion) so as to induce CO₂ pressurization of the system including that of the second liquid component. When the nozzle is depressed again the two liquid components are forced to mix together in the mixing assembly the resultant flash-dry disinfectant composition is expelled out through the spray nozzle as a spray aerosol.

The various elements or components of the present invention such as the materials for the flash-dry disinfectant composition (including their proportions in the flash-dry disinfection composition), the components or element of the aerosol apparatus (i.e. the spray nozzle head) are of curse to be selected with a view to the desired or.necessary ends herein i.e. disinfection/drying.

The surface(s) to be treated in accordance with the present invention may be hard (e.g. metal) or soft (e.g. carpet)

The following examples illustrate various example flash-dry disinfectant compositions of the present. The various microrganisms used for the tests as well as the procedure (protocol 4) used to effect various tests will be discussed after the examples.

### Example 1

| **EVAPORATION TIME FOR VARIOUS AEROSOL STERILIZER** | | | | | | | |
|---|---|---|---|---|---|---|---|
| **Solutions** | **Evaporation Time** | | | | | | |
| | 1 minute | 2 minutes | 5 minutes | 15 minutes | 30 minutes | 40 minutes | 45 minutes |
| H₂O | wet | wet | humid | droplets | droplets | droplets | dry |
| H₂O₂ 10% | wet | wet | humid | driplets | droplets | droplets | dry |
| H₂O₂ 10% in ETOH | wet | humid | dry | dry | dry | dry | dry |
| ETOH 95% | humid | dry | dry | dry | dry | dry | dry |

### Example 2

The sterilizing effect of an aerosol mixture containing H₂O₂/ETOH on various materials inoculated with BG for 60 minutes contact time.
Temperature: 20.7°C

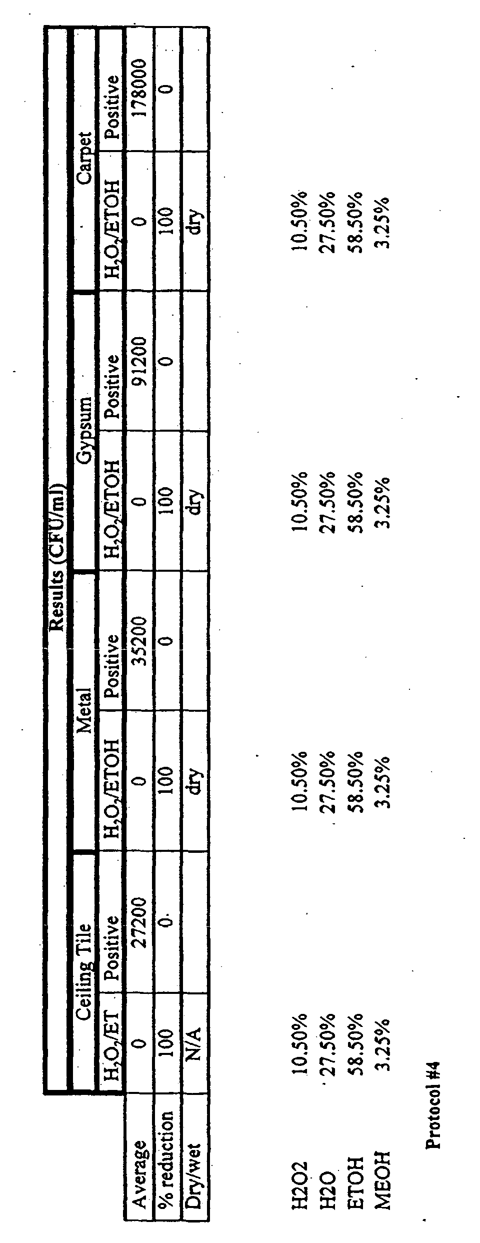

### Example 3

The comparative performance of a sterilizing agent against MS2 phage on various surfaces for 60 minutes contact time.
Temperature: 22.4°C

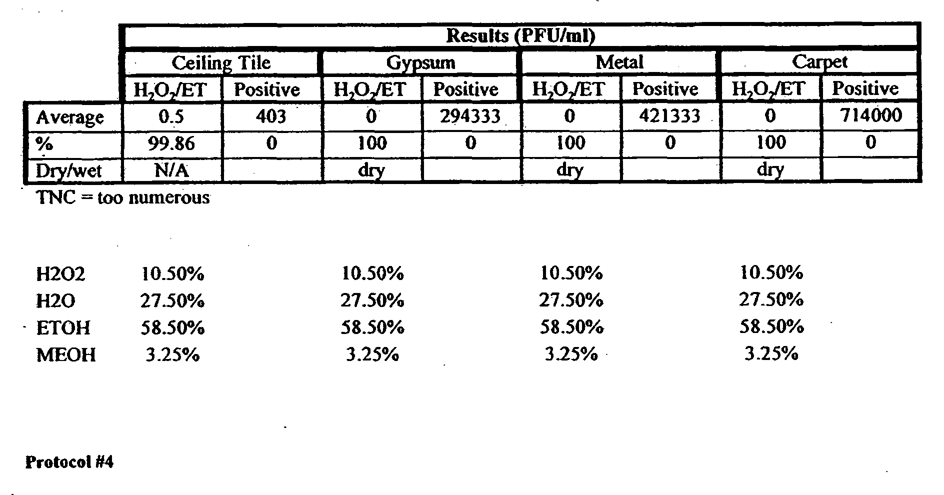

### Example 4

Sterilizing effect of H₂O₂ 10% in Ethanol (lot 991208) on Gypsum wallboard pieces inoculated with BG spores.
Temperature: 19.6°C

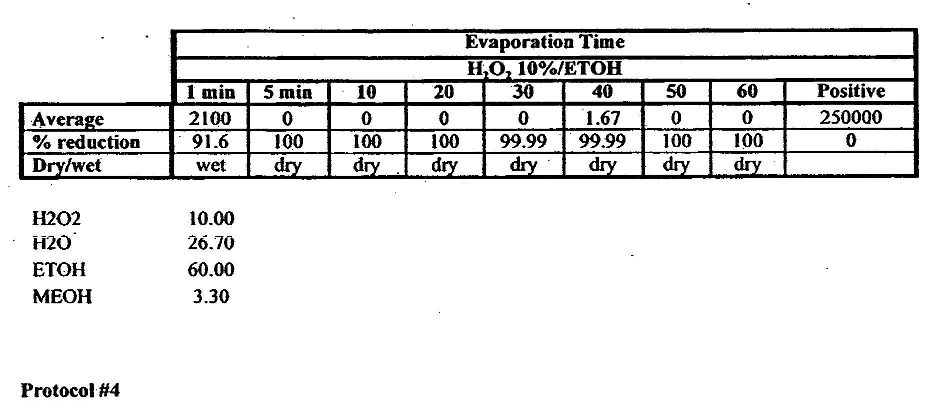

### Example 5

Performance of H₂O₂ in ETOH against BG spores on gypsum board places for 60 minutes contact time.
Temperature: 19.6°C

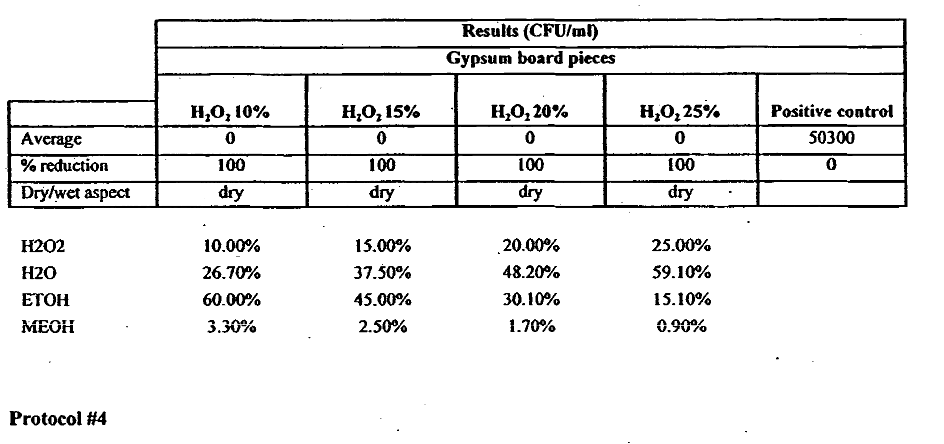

### Example 6

Performance of H₂O₂ 10% in methanol against BG spores on gypsum board pieces for different contact time
Temperature: 19.0°C

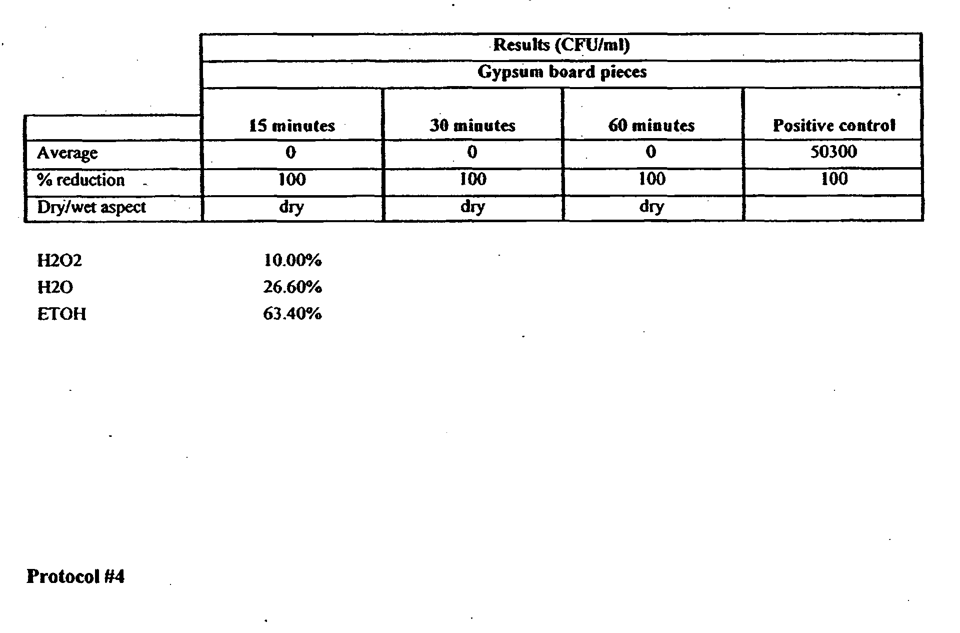

### Example 7

Performance of H₂O₂10% in Isopropyl alcohol against BG spores on gypsum board pieces for different contact time
Temperature: 19.0°C

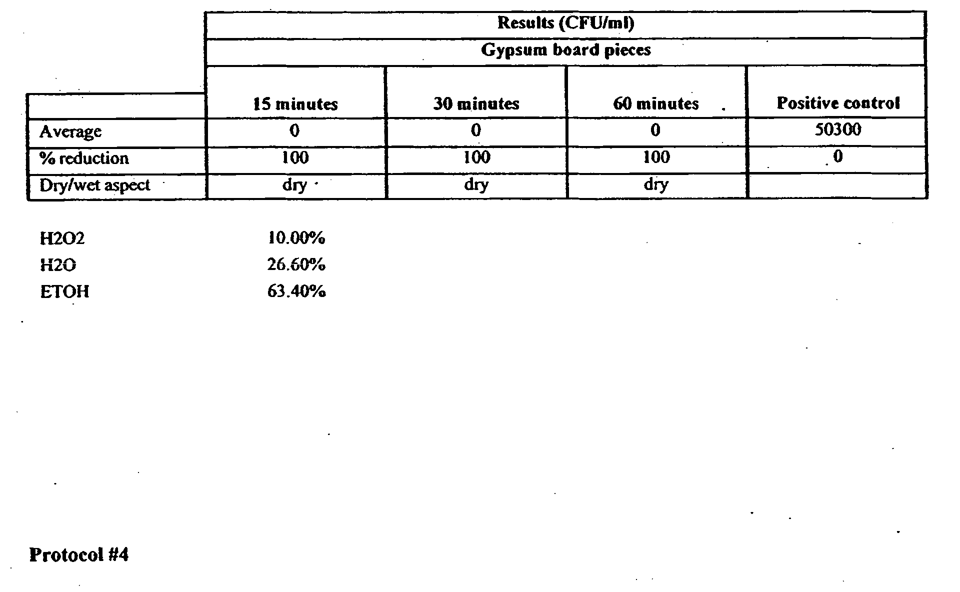

### Example 8

Performance of H₂O₂ 10% in propanol against BG spores on gypsum board pieces for different contact time
Temperature: 19.8°C

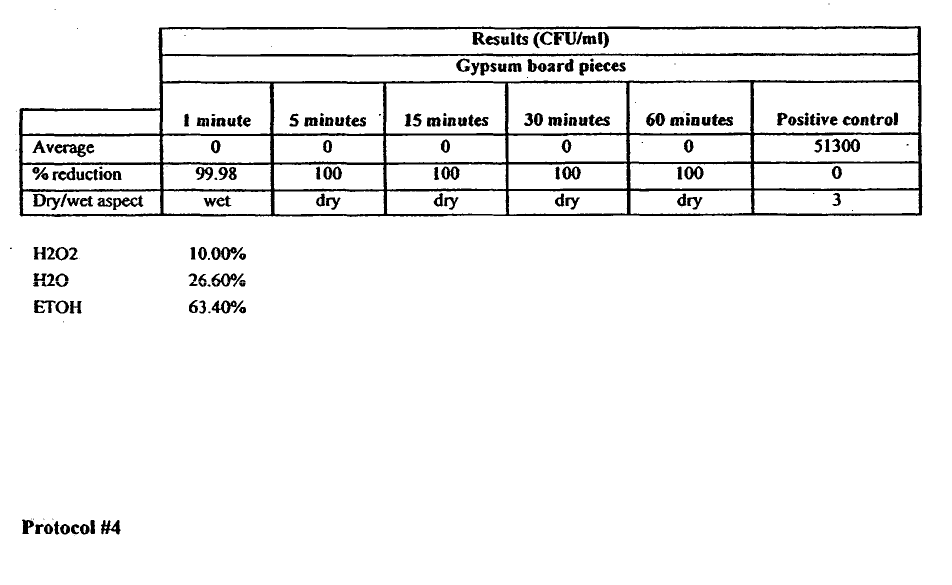

### Example 9

Performance of H₂O₂ 10% in isopropyl alcohol against BG spores on gypsum board pieces for different contact time
Temperature: 19.8°C

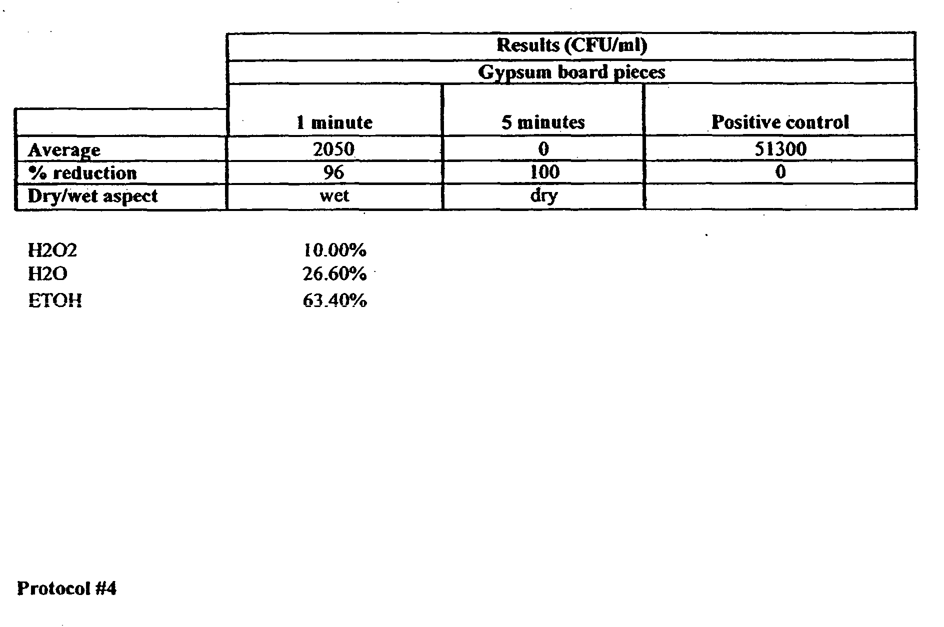

### Example 10

Performance of H₂O₂10% in methanol against BG spores on gypsum board pieces for different contact time
Temperature: 19.8°C

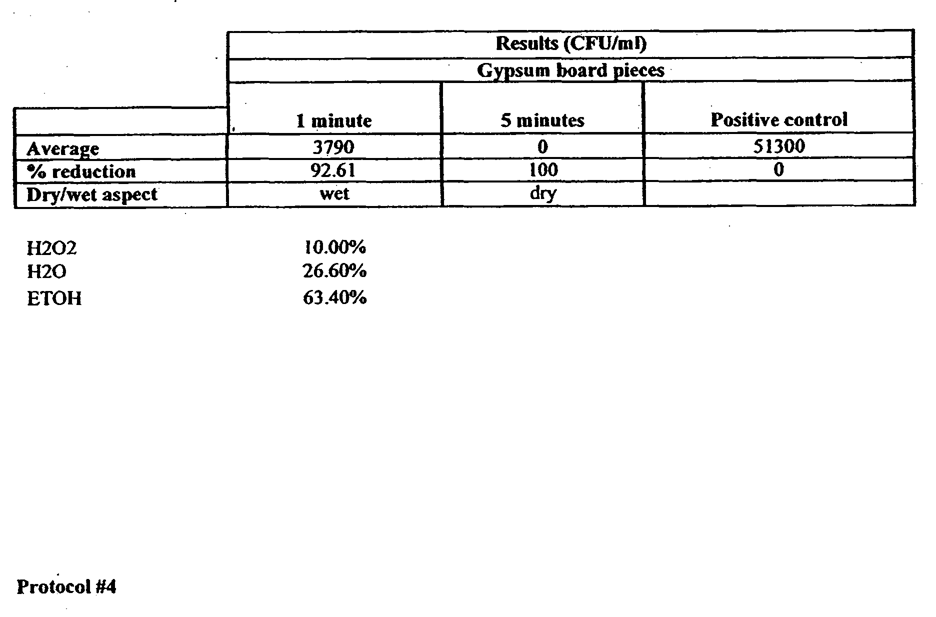

### Bacillus subtilis var niger spores (BG)

*Bacillus subtilis* var niger spores (ATCC.9372) is used as challenge organism. BG is a Gram-positive spore-forming bacteria that is widely used as an accepted surrogate for spore-forming bacterial BW agents such as *Bacillus anthracis*. The extreme protection that sporulation gives to this mircroorganism makes it one of the most difficult to eliminate. BG spores are produced following our internal Work Instructions based on standard microbiology procedures.

### MS2 coliphage

The other challenge organism used for the sterilizing tests is MS2 (ATCC 15597-B 1). MS2 is an approximately 26 nm icosahedral bactcriophage that infects *Escherichia coli* (ATCC 15597). It is an accepted surrogate for vital BW agents, including Ebola and Venezuelan Equine Encephalitis. MS2 is prepared according to Dugway Proving Ground SOP.

### Protocol #4

### Apparatus and Reagents

Gypsum wallboard piece of 10cm*10cm
Carpet piece of 10cm*10cm
Sheet metal piece of 10cm*10cm
Ceiling tile piece of 10cm*10cm
Tissue of partition panels piece of 10cm*10cm
NDS buffer
Incubator
*Bacillus subtilis* var niger spores and MS2 phage
Spray can (Sur Shot Atomizer, Model B sprayer, Milwaukee Sprayer MFG. CO. INC., Milwaudee, WI, USA) with fin spray nozzle (#B30-040) filled with biocidal solutions and air or gaseous nitrogen.

### Procedure #4

1. With a sponge, spread the inoculum at a concentration of 10⁷ CFU/ml or PFU/ml over the clean surface of the pieces to be tested. Keep 1 cm border free of microorganisms.
2. Using the spray can, treat all the surface of the pieces for 3 to 5 seconds.
3. Wait for the chosen contact time.
4. Sample the experimental and positive control pieces of the gypsum board, sheet metal and ceiling tile with a swab in 9 ml of NDS buffer. Sample the experimental and positive control pieces of the carpet and tissue, by soaking the pieces in 27 ml of NDS buffer and then sampling 1 ml of the residual liquid.
5. For BG spores, place 0.2 ml of each dilution on TSA media; for MS2 phage, place 1.0 ml of each dilution on MS2 media. Incubate at 35°C for 24 hours.

### Results and discussion

Numerous experiments were performed as to evaluate the comparative performance of TAS against MS2 phage and BG spores under different environmental conditions.

The Neutralizing Dilution Solution (NDS buffer) used for purpose of sampling contains sodium thiosulfate, which is the agent responsible for the neutralization of the oxidants (such as hydrogen peroxide) present in the experimental samples. The neutralization action of sodium thiosulfate prevents the oxidizing process from going on once the sampling has occurred, ensuring the accuracy of the chosen contact time.

The sterilizing solution is applied by means of an aerosol spray can (Sure Shot Atomizer, Model B sprayer, Milwaukee Sprayer MFG. CO. INC., Milwaukee, WI, USA) filled with the biocidal solution and air or gaseous nitrogen (see Joe's scheme). The gas propellant ensures the propulsion in the form of an aerosol of the sterilizing solution to be expelled. Adapted spray nozzles come in different sizes. We used the finest nozzle available (#B30-040) in order to produce an aerosol with droplet size as small as possible. The physico/chemical properties of the biocidal agents combined with the aerosol application method contribute to generate a flash-dry sterilizing solution.

Microbiological results are presented in the Examples 1 to 10. Prior experimentation proved the concentration of 10% of hydrogen peroxide in combination with ethanol (ETOH) to provide a 100% sterilizing efficacy as well as a short evaporation time. The performance of hydrogen peroxide 10% (H₂O₂ 10%) in combination with different alcohols against BG spores on gypsum board pieces was evaluated for various contact time throughout Examples 6 to 10. Positive results were observed for all the alcohols tested (isopropyl alcohol, methanol and propanol all in a concentration of 63,4%) and all the configurations with 5-minute contact time and above presented a 100% sterilizing efficacy. After 5 minutes of contact time all sample surfaces were left dry. It is worth noting that after 1 minute of contact time, an average of 96, 1 % of microorganisms are already eliminated.

The sterilizing efficacy of the hydrogen peroxide/ethanol solution was tested on various surfaces in order to assess its efficacy on different materials Examples 2 and 3. The solution was challenged with MS2 and BG for 60 minutes of contact time. We obtained reduction rates of a 100% for all the samples with the exception of the ceiling tile sample in Example 3 that yielded a 99,86% result.

The selected sterilizing solution (H₂O₂ 10% in ethanol) is a flash-dry biocidal solution with high and fast kill rate. The said solution combines a 100% sterilizing efficacy against MS2 and BG spores within 5 minutes of contact time with flash-dry properties.

## Claims

1. In an aerosol spray apparatus, for applying an antimicrobial agent to a surface to be disinfected, comprising an aerosol spray nozzle coupled to a container means comprising gas propellant means and means for containing a liquid disinfectant composition able to be sprayed from the apparatus, said aerosol apparatus being configured for dispensing said disinfectant composition through said spray nozzle, under pressure due to the presence of the gas propellant means, as an aerosol spray onto said surface to be disinfected, the improvement wherein the liquid disinfectant composition is a liquid flash-dry disinfectant composition comprising a flash vaporisation component and an effective amount of an antimicrobial agent, said flash vaporisation component being able, once the flash-dry disinfectant composition is sprayed from the apparatus, to flash vaporise so as to leave an essentially dry surface having antimicrobial agent deposited thereon.

2. An aerosol apparatus as defined in claim 1 wherein said liquid flash-dry disinfectant composition consists of said flash vaporisation component and said antimicrobial agent.

3. An aerosol apparatus as defined in claim 1 or 2 wherein said antimicrobial agent comprises hydrogen peroxide.

4. An aerosol apparatus as defined in claim 1 or 2 wherein said flash vaporisation component comprises ethanol.

5. An aerosol apparatus as defined in claim 1 wherein the flash-dry disinfectant composition comprise 3 to 30% by volume hydrogen peroxide, 10 to 50 % by volume of ethanol and 10 to 65 % by volume of water.

6. A liquid flash-dry aerosol disinfectant composition comprising a flash vaporisation component and an effective amount of an antimicrobial agent, said flash vaporisation component being able, once the flash-dry disinfectant composition is sprayed in aerosol form onto a surface, to flash vaporise so as to leave an essentially dry surface having

7. A liquid flash-dry aerosol disinfectant composition as defined in claim 6 wherein said liquid flash-dry disinfectant composition consists of said flash vaporisation component and said antimicrobial agent.

8. A liquid flash-dry aerosol disinfectant composition as defined in claim 6 wherein said antimicrobial agent comprises hydrogen peroxide.

9. A liquid flash-dry aerosol disinfectant composition as defined in claim 6 wherein said flash vaporisation component comprises ethanol.

10. A liquid flash-dry aerosol disinfectant composition as defined in claim 6 wherein the flash-dry disinfectant composition comprises 3 to 30% by volume hydrogen peroxide, 10 to 85 % by volume of ethanol and 10 to 65 % by volume of water.

11. A method for disinfecting a surface comprising applying a liquid flash-dry disinfectant composition as an aerosol spray onto such surface, said liquid flash-dry aerosol disinfectant composition comprising a flash vaporisation component and an effective amount of an antimicrobial agent, said flash vaporisation component being able, once the flash-dry disinfectant composition is sprayed in aerosol form onto a surface, to flash vaporise so as to leave an essentially dry surface having antimicrobial agent deposited thereon.

12. A method as defined in claim 11 wherein said liquid flash-dry disinfectant composition consists of said flash vaporisation component and said antimicrobial agent.

13. A method as defined in claim 11 wherein said antimicrobial agent comprises hydrogen peroxide.

14. A method as defined in claim 11 wherein said flash vaporisation component comprises ethanol.

15. A method as defined in claim 11 wherein the flash-dry disinfectant composition comprises 3 to 30% by volume hydrogen peroxide, 10 to 85 % by volume of ethanol and 10 to 65 % by volume of water.

16. A method as defined in claim 11 wherein said antimicrobial agent and said flash vaporisation component are mixed together just prior to said liquid flash-dry aerosol disinfectant composition being applied to said surface.

17. In an aerosol spray apparatus, for applying an antimicrobial agent to a surface to be disinfected, comprising an aerosol spray nozzle coupled to a container means comprising gas propellant means, said aerosol apparatus being configured for dispensing a liquid disinfectant composition through said spray nozzle, under pressure due to the presence of the gas propellant means, as an aerosol spray onto said surface to be disinfected, the improvement wherein said liquid disinfectant composition is a liquid flash-dry disinfectant composition able to be sprayed from said apparatus, said liquid flash-dry disinfectant composition comprising a flash vaporisation component and an effective amount of an antimicrobial agent element, said flash vaporisation component being able, once the flash-dry disinfectant composition is sprayed from the apparatus, to flash vaporise so as to leave an essentially dry surface having antimicrobial agent deposited thereon, wherein said container means comprises a first container means containing said antimicrobial agent and a second container means containing said flash vaporisation component and wherein said aerosol spray apparatus comprises mixing means for mixing said antimicrobial agent and said flash vaporisation component together prior to dispensing said liquid flash-dry disinfectant composition through said spray nozzle.

18. An aerosol apparatus as defined in claim 17 wherein said liquid flash-dry disinfectant composition consists of said flash vaporisation component and said antimicrobial agent.

19. An aerosol apparatus as defined in claim 17 wherein said antimicrobial agent comprises hydrogen peroxide.

20. An aerosol apparatus as defined in claim 17 wherein said flash vaporisation component comprises ethanol.

21. An aerosol apparatus as defined in claim 17 wherein the flash-dry disinfectant composition comprises 3 to 30% by volume hydrogen peroxide, 10 to 85 % by volume of ethanol and 10 to 65 % by volume of water.
